# EUROPÄISCHE PATENTSCHRIFT

(11) **EP 0 770 598 B1**
(45) Veröffentlichungstag und Bekanntmachung des Hinweises auf die Patenterteilung: **25.07.2001**
(21) Anmeldenummer: 96116473.8
(22) Anmeldetag: 15.10.1996
(51) Int. Cl.: C07C 281/02

(54) **Verfahren zur Herstellung von Carbazaten**
Process for the preparation of carbazates
Procédé pour la préparation de carbazates

(30) Priorität: 27.10.1995 DE 19540073
(43) Veröffentlichungstag der Anmeldung: 02.05.1997
(73) Patentinhaber: BAYER AG, 51368 Leverkusen (DE)
(72) Erfinder: Landscheidt, Heinz, Dr., 47057 Duisburg (DE); Ritzer, Edwin, Dr., 51381 Leverkusen (DE); Klausener, Alexander, Dr., 50670 Köln (DE)

(56) Entgegenhaltungen:
- EP-A- 0 103 400
- JOURNAL OF THE AMERICAN CHEMICAL SOCIETY, Bd. 70, März 1948, Seiten 1181-1183, XP000617257 RABJOHN N: "THE SYNTHESIS AND REACTIONS OF DISAZODICARBOXYLATES"
- BERICHTE DER DEUTSCHEN CHEMISCHEN GESELLSCHAFT, Bd. 47, Nr. 2, 1914, Seiten 2183-2188, XP000617234 DIELS O: "DARSTELLUNG UND NEUE REAKTIONEN DER HYDRAZIN-MONOCARBONSAEUREESTER"
- JOURNAL OF BIOLOGICAL CHEMISTRY, Bd. 266, Nr. 9, 25.März 1991, Seiten 5525-5533, XP000616569 SCAMAN C H ET AL: "INHIBITION OF CYTOPLASMIC ASPARTATE AMINOTRANSFERASE FROM PORCINE HEART BY R AND S ISOMERS OF AMINOOXYSUCCINATE AND HYDRAZINOSUCCINATE"

## Beschreibung

Die vorliegende Erfindung betrifft ein Verfahren zur Herstellung von gegebenenfalls substituierten Carbazaten durch Umsetzung von unsubstituierten C₁-C₄-Alkylcarbazaten mit gegebenenfalls substituierten Alkoholen in Gegenwart eines Katalysators.

Carbazate werden als Zwischenprodukte zur Herstellung von Pflanzenschutzmitteln und Pharmazeutika eingesetzt. Sie werden insbesondere zu Peptidsynthesen verwendet (siehe EP-OS 106 282). Nach J. Biol. Chem. 266, 5525 (1991) läßt sich aus Benzylcarbazat Hydrazino-succinat, ein Inhibitor der Aspartat-Aminotransferase, herstellen. Die EP-OS 143 078 beschreibt den Einsatz von Benzylcarbazat zur Herstellung von Pflanzenschutzmitteln.

Carbazate werden im allgemeinen durch Umsetzung von Chlorameisensäureestern mit Hydrazin gewonnen. So wird in Chem. Ber. 92, 1478 (1959) die Reaktion von Chlorameisensäurebenzylester mit Hydrazin unter Bildung von Benzylcarbazat beschrieben. Hierbei ist jedoch die Herstellung der Vorprodukte, die unter Verwendung von Phosgen erfolgt, vergleichsweise aufwendig.

Nach einer anderen Methode werden symmetrische Dialkylcarbonate mit Hydrazin umgesetzt (siehe J. Am. Chem. Soc. 70, 1181 (1948)). Auch hier ist die Herstellung der Vorprodukte, vor allem, wenn es sich dabei um höhere Carbonate handelt, schwierig oder unmöglich. Dibenzylcarbonat läßt sich beispielsweise nur in sehr unreiner Form erhalten, da dann stets Benzylchlorid als Nebenprodukt auftritt. Diese Verfahrensweise ist auch wenig wirtschaftlich, da der als Nebenprodukt anfallende Alkohol entweder entsorgt oder durch einen zusätzlichen Isolierschritt wiedergewonnen werden muß.

Es besteht also noch ein Bedürfnis nach einem Verfahren zur sicheren, einfach durchführbaren und ökonomischen Herstellung von Carbazaten.

Es wurde nun ein Verfahren zur Herstellung von Carbazaten der Formel (I) gefunden

R¹-O-CO-NH-NH₂ (I),

in der R¹ für geradkettiges oder verzweigtes C₅-C₁₈-Alkyl, das gegebenenfalls mit Fluor, Chor, der Gruppe X-R³ (mit X = Sauerstoff oder Schwefel und R³ = geradkettigem oder verzweigtem C₁-C₄-Alkyl), für gegebenenfalls substituiertes C₃-C₆-Cycloalkyl, für gegebenenfalls substituiertes C₆-C₁₂-Aryl, für C₂-C₁₈-Alkyl, das mit Phenyl substituiert ist, für Benzyl oder für Cyclopropylmethyl steht,
das dadurch gekennzeichnet ist, daß man ein Alkylcarbazat der Formel (II)

R²-O-CO-NH-NH₂ (II),

in der R² für geradkettiges oder verzweigtes, unsubstituiertes C₁-C₄-Alkyl steht,
mit Alkoholen der Formel (III)

R¹-OH (III),

in der R¹ wie bei Formel (I) angegebene Bedeutung hat,
in Gegenwart eines Katalysators umsetzt.

Geradkettige und verzweigte unsubstituierte C₁-C₄-Alkylcarbazate der Formel (II) sind im großtechnischem Maßstab durch Umsetzung von Dialkylcarbonaten der Formel (IV)

R²-O-CO-O-R² (IV),

in der R² die bei Formel (II) angegebene Bedeutung hat,
mit Hydrazin gut zugänglich (siehe z.B. Ber. Chem. Ges. 47, 2183 bis 2188 (1914). Dimethylcarbonat ist auch im großtechnischen Maßstab ohne Einsatz von Phosgen herzustellen, beispielsweise durch Oxidation von Kohlenmonoxid mit Sauerstoff in Gegenwart von Methanol und Katalysatoren (siehe z.B. EP-OS 365 083).

Der bei dieser Reaktion freigesetzte Alkohol kann aufgefangen werden, so daß die erfindungsgemäße Reaktion insgesamt ohne aufwendigen Chemikalienbedarf und ohne die Produktion größerer Mengen an Abfällen durchgeführt werden kann.

Soweit in den Formeln (I) und (III) R¹ substituiertes C₃-C₆-Cycloalkyl oder substituiertes C₆-C₁₂-Aryl bedeutet, kommen als Substituenten beispielsweise infrage: Halogen, Hydroxy, die Gruppe X-R³ (mit X = Sauerstoff oder Schwefel und R³ = geradkettiges oder verzweigtes C₁-C₄-Alkyl), die Gruppe COOR⁴ (mit R⁴ = C₁-C₄-Alkyl), die Gruppe NR⁵R⁶ (mit R⁵ und R⁶ = unabhängig voneinander jeweils C₁-C₄-Alkyl) und gegebenenfalls mit geradkettigen oder verzweigten C₁-C₄-Alkylgruppen substituiertes C₃-C₆-Cycloalkyl. Soweit in den Formeln (I) und (III) R¹ substituiertes C₃-C₆-Cycloalkyl bedeutet, kommen als Substituenten auch gegebenenfalls substituiertes C₆-C₁₄-Aryl und gegebenenfalls substituiertes C₆-C₁₄-Aryloxy, 1-Piperidinyl, 1-Morpholinyl und 1-Pyrrolidinyl in Frage.

Bei den Arylresten, auch in Form von Substituenten für Cycloalkyl und in Form von Aryloxy-Substituenten, handelt es sich vorzugsweise um Phenyl, Naphthyl oder Biphenyl.

Als Substituenten für Aryl- und Aryloxyreste kommen z.B. C₁-C₄-Alkyl, C₁-C₄-Alkoxy, Di-C₁-C₄-Alkylamino, Nitro und Halogen infrage.

Alle genannten Substituenten können einfach oder mehrfach vorhanden sein. Es können auch zwei oder mehr unterschiedliche Substituenten nebeneinander vorliegen.

Soweit R¹ für gegebenenfalls substituiertes C₃-C₆-Cycloalkyl steht, sind als Substituenten Fluor, Chlor, die Gruppe X-R³ (wie oben definiert), oder gegebenenfalls mit C₁-C₄-Alkyl, C₁-C₄-Alkoxy, Fluor und/oder Chlor substituiertes Phenyl bevorzugt.

Besonders bevorzugt bedeutet R¹ geradkettiges C₅-C₁₈-Alkyl, das gegebenenfalls mit Phenyl substituiert ist oder C₃-C₆-Cycloalkyl, das gegebenenfalls mit C₁-C₂-Alkyl substituiert ist.

Ganz besonders bevorzugt bedeutet R¹ Pentyl, i-Pentyl, Hexyl, Dodecyl, Hexadecyl, Octadecyl, Cyclopropyl, Cyclobutyl, Cyclopentyl oder Cyclohexyl.

In den Formeln (II) und (IV) steht R² vorzugsweise für Methyl oder Ethyl.

Zur Durchführung des erfindungsgemäßen Verfahrens kann das jeweilige Alkylcarbazat der Formel (II), beispielsweise Methylcarbazat, mit dem jeweiligen Alkohol der Formel (III) z.B. in einem Molverhältnis von 0,5 bis 20:1, bevorzugt 0,9 bis 5:1, ganz besonders bevorzugt 0,9 bis 1,5:1, gegebenenfalls unter Zusatz eines unter den Reaktionsbedingungen stabilen Lösungsmittels umgesetzt werden. Als Lösungsmittel kommen z.B. Kohlenwasserstoffe, Ether und Säureamide in Frage - wie Cyclohexan, Toluol, Chlorbenzol, Ethylen- und Diethylenglykoldimethylether und Dimethylformamid. Vorteilhafterweise setzt man rohes Alkylcarbazat der Formel (II) ein wie es beispielsweise bei der Umsetzung von Dialkylcarbonaten der Formel (IV) mit Hydrazin ohne weitere Reinigung oder Zwischenisolierung erhalten wird.

Als Katalysatoren kommen die vielfältigsten basisch reagierenden Verbindungen infrage. Aus Gründen der erleichterten Abtrennbarkeit nach Beendigung des erfindungsgemäßen Verfahrens sind feste basische Verbindungen als Katalysator bevorzugt. Beispiele für Katalysatoren sind: Alkalimetallhydroxide wie Lithiumhydroxid, Natriumhydroxid und Kaliumhydroxid, Erdalkalimetallhydroxide wie Calciumhydroxid, Alkalimetall- und Erdalkalimetallcarbonate und -hydrogencarbonate wie Lithiumcarbonat, Natriumcarbonat, Kaliumcarbonat, Magnesiumcarbonat, Calciumcarbonat, Natriumhydrogencarbonat und Kaliumhydrogencarbonat und Alkalimetall- und Erdalkalimetallalkoholate wie Lithiummethanolat und Natriummethanolat. Bei Verwendung von Alkalimetall- und Erdalkalimetallalkoholaten wählt man zur Vermeidung der Bildung unerwünschter Nebenprodukte bevorzugt solche aus, die sich vom jeweils eingesetzten Alkohol der Formel (III) oder vom als Nebenprodukt gebildeten Alkohol der Formel R²-OH ableiten.

Ebenfalls als Katalysatoren einsetzbar sind Aminoverbindungen, beispielsweise bicyclische Aminoverbindungen wie 1,5-Diazabicyclo[4.3.0]non-5-en und 1,8-Diazabicyclo[5.4.0]undec-7-en, Titanverbindungen wie Titan (IV)-isopropylat und Zinnverbindungen wie Dibutylzinnoxid und Dimethylzinndidodecanat.

Der Katalysator kann z.B. in Mengen von 0,001 bis 10 Gew.-%, bezogen auf eingesetztes Alkylcarbazat der Formel (II), eingesetzt werden.

Die erfindungsgemäße Umsetzung kann beispielsweise bei Temperaturen von 50 bis 250°C, bevorzugt 80 bis 180°C, und bei einem Druck von 0,1 bis 10 bar, vorzugsweise Normaldruck durchgeführt werden.

Der im Verlaufe der erfindungsgemäßen Umsetzung gebildete Alkohol der Formel R²-OH wird bevorzugt bereits während der Umsetzung abdestilliert. Dieser Vorgang kann gegebenenfalls durch Zusatz eines Azeotropbildners, etwa eines aromatischen Kohlenwasserstoffs, insbesondere Toluol, gefördert werden.

Nach Beendigung der erfindungsgemäßen Umsetzung kann man das Reaktionsgemisch beispielsweise aufarbeiten, indem man den Katalysator abtrennt, beispielsweise durch Filtration oder durch Extraktion mit Wasser. Eventuell vorhandener überschüssiger Alkohol kann gegebenenfalls durch Destillation abgetrennt werden. Das verbleibende rohe Reaktionsprodukt kann gegebenenfalls mit Hilfe an sich bekannter Techniken, z.B. durch Destillation oder Kristallisation weiter gereinigt werden.

Es ist ausgesprochen überraschend, daß es mit dem erfindungsgemäßen Verfahren gelingt auf technisch einfache Weise und unter Vermeidung von schwierig zu handhabenden Chemikalien und Abfällen Carbazate herstellen, insbesondere solche mit größeren und/oder komplizierter gebauten Resten an der Carbonyloxygruppe. Aus dem Stand der Technik ist nämlich bekannt, daß sich in Nachbarschaft zu Carbonylgruppen befindliche Aminogruppen (eine Anordnung wie sie auch bei den Ausgangsverbindungen der Formel (I) für die erfindungsgemäße Umsetzung vorliegt) mit Alkoholen unter Bildung von Urethanen oder Kohlensäureestern umsetzen (siehe z.B. US-PS 2 834 799, US-PS 2 837 561 und EP-OS 13 957).

### Beispiele

### Beispiel 1

45 g Methylcarbazat wurden mit 54,0 g Benzylalkohol und 2,0 g Kaliumcarbonat vermischt. Die so erhaltene Mischung wurde 3 Stunden lang auf 130°C erhitzt. Das dabei entstehende Methanol wurde abdestilliert. Zur Aufarbeitung des Reaktionsgemisches wurde der Katalysator nach Abkühlung auf Raumtemperatur durch - Filtration abgetrennt und der übrige Benzylalkohol abdestilliert. Der Rückstand (46,5 g) bestand nach gaschromatografischer Analyse zu 97 Gew.-% aus Benzylcarbazat. Der Schmelzpunkt des Produkts betrug 69°C, das ¹H-NMR-Spektrum zeigte, gemessen in Deuterochloroform, charakteristische Absorptionen bei δ = 3,5; 5,15; 6,35 und 7,25 - 7,4 ppm.

## Patentansprüche

1. Verfahren zur Herstellung von Carbazaten der Formel (I)
R¹-O-CO-NH-NH₂ (I),
in der R¹ für geradkettiges oder verzweigtes C₅-C₁₈-Alkyl, das gegebenenfalls mit Fluor, Chlor, der Gruppe X-R³ (mit X = Sauerstoff oder Schwefel und R³ = geradkettigem oder verzweigtem C₁-C₄-Alkyl), für gegebenenfalls substituiertes C₃-C₆-Cycloalkyl, für gegebenenfalls substituiertes C₆-C₁₂-Aryl, für C₂-C₁₈-Alkyl, das mit Phenyl substituiert ist, für Benzyl oder für Cyclopropylmethyl steht,
dadurch gekennzeichnet, daß man ein Alkylcarbazat der Formel (II)
R²-O-CO-NH-NH₂ (II),
in der R² für geradkettiges oder verzweigtes, unsubstituiertes C₁-C₄-Alkyl steht,
mit einem Alkohol der Formel (III)
R¹-OH (III),
in der R¹ die bei Formel (I) angegebene Bedeutung hat,
in Gegenwart eines Katalysators umsetzt.

2. Verfahren nach Anspruch 1, dadurch gekennzeichnet, daß in den Formeln (I) und (III) R¹ soweit es für substituiertes C₃-C₆-Cycloalkyl oder substituiertes C₆-C₁₂-Aryl steht, als Substituenten Halogen, Hydroxy, die Gruppe X-R³ (mit X = Sauerstoff oder Schwefel und R³ = geradkettiges oder verzweigtes C₁-C₄-Alkyl), die Gruppe COOR⁴ (mit R⁴ = C₁-C₄-Alkyl), die Gruppe NR⁵R⁶ (mit R⁵ und R⁶ = unabhängig voneinander jeweils C₁-C₄-Alkyl), und gegebenenfalls mit geradkettigen oder verzweigten C₁-C₄-Alkylgruppen substituiertes C₃-C₆-Cycloalkyl und soweit es für substituiertes C₃-C₆-Cycloalkyl steht als Substituenten auch gegebenenfalls substituiertes C₆-C₁₄-Aryl oder gegebenenfalls substituiertes C₆-C₁₄-Aryloxy, 1-Piperidinyl, 1-Morpholinyl und 1-Pyrrolidinyl, enthält.

3. Verfahren nach Ansprüchen 1 und 2, dadurch gekennzeichnet, daß R¹ für C₃-C₆-Cycloalkyl steht, das gegebenenfalls mit Fluor, Chlor, der Gruppe X-R³ (wie in Anspruch 2 definiert), oder gegebenenfalls mit C₁-C₄-Alkyl, C₁-C₄-Alkoxy, Fluor und/oder Chlor substituiertem Phenyl substituiert ist.

4. Verfahren nach Ansprüchen 1, dadurch gekennzeichnet, daß R¹ für C₃-C₆-Cycloalkyl, das gegebenenfalls mit C₁-C₂-Alkyl substituiert ist, steht.

5. Verfahren nach Ansprüchen 1 bis 4, dadurch gekennzeichnet, daß R¹ für Pentyl, i-Pentyl, Hexyl, Dodecyl, Hexadecyl, Octadecyl, Cyclopropyl, Cyclobutyl, Cyclopentyl, Cyclohexyl steht.

6. Verfahren nach Ansprüchen 1 bis 5, dadurch gekennzeichnet, daß man als Alkylcarbazat der Formel (II) solches einsetzt, bei dem R² für Methyl oder Ethyl steht.

7. Verfahren nach Ansprüchen 1 bis 6, dadurch gekennzeichnet, daß man ein Alkylcarbazat der Formel (II) und einen Alkohol der Formel (III) in einem Molverhältnis von 0,5 bis 20:1 einsetzt.

8. Verfahren nach Ansprüchen 1 bis 7, dadurch gekennzeichnet, daß man als Katalysatoren Alkalimetallhydroxide, Erdalkalimetallhydroxide, Alkali- oder Erdalkalimetallcarbonate oder -hydrogencarbonate oder Alkalimetall- oder Erdalkalimetallalkoholate, Aminoverbindungen, Titanverbindungen oder Zinnverbindungen einsetzt.

9. Verfahren nach Ansprüchen 1 bis 8, dadurch gekennzeichnet, daß man rohe Alkylcarbazate der Formel (II) einsetzt, wie sie bei der Umsetzung von Dialkylcarbonaten der Formel (IV)
R²-O-CO-O-R² (IV),
in der R² die in Anspruch 2 bei Formel (II) angegebene Bedeutung hat, mit Hydrazin ohne weitere Reinigung oder Zwischenisolierung erhältlich sind.

10. Verfahren nach Ansprüchen 1 bis 9, dadurch gekennzeichnet, daß der Katalysator in Mengen von 0,001 bis 10 Gew.-%, bezogen auf das Alkylcarbazat der Formel (II) eingesetzt wird und die Umsetzung bei 50 bis 250°C durchgeführt wird.

## Claims

1. Process for the preparation of carbazates of the formula (I)
R¹-O-CO-NH-NH₂ (I),
in which R¹ represents straight-chain or branched C₅-C₁₈-alkyl which is optionally substituted by fluorine, chlorine, the group X-R³ (where X = oxygen or sulphur and R³ = straight-chain or branched C₁-C₄-alkyl), optionally substituted C₃-C₆-cycloalkyl, optionally substituted C₆-C₁₂-aryl, C₂-C₁₈-alkyl which is substituted by phenyl, benzyl or cyclopropyl,
characterized in that an alkyl carbazate of the formula (II)
R²-O-CO-NH-NH₂ (II),
in which R² represents straight-chain or branched, unsubstituted C₁-C₄-alkyl,
is reacted with an alcohol of the formula (III)
R¹-OH (III),
in which R¹ is as defined for formula (I),
in the presence of a catalyst.

2. Process according to Claim 1, characterized in that, in the formulae (I) and (III), R¹ as substituted C₃-C₆-cycloalkyl or substituted C₆-C₁₂-aryl contains as substituents halogen, hydroxyl, the group X-R³ (where X = oxygen or sulphur and R³ = straight-chain or branched C₁-C₄-alkyl), the group COOR⁴ (where R⁴ = C₁-C₄-alkyl), the group NR⁵R⁶ (where R⁵ and R⁶ = independently of one another in each case C₁-C₄-alkyl), and C₃-C₆-cycloalkyl which is optionally substituted by straight-chain or branched C₁-C₄-alkyl groups and, as substituted C₃-C₆-cycloalkyl, contains as substituents, in addition, optionally substituted C₆-C₁₄-aryl or optionally substituted C₆-C₁₄-aryloxy, 1-piperidinyl, 1-morpholinyl and 1-pyrrolidinyl.

3. Process according to Claim 1, characterized in that R¹ represents C₃-C₆-cycloalkyl which is optionally substituted by fluorine, chlorine, the group X-R³ (as defined in claim 2) or optionally C₁-C₄-alkyl-, C₁-C₄-alkoxy-, fluorine- and/or chlorine-substituted phenyl.

4. Process according to Claim 1, characterized in that R¹ represents C₃-C₆-cycloalkyl which is optionally substituted by C₁-C₂-alkyl.

5. Process according to Claims 1 to 4, characterized in that R¹ represents pentyl, i-pentyl, hexyl, dodecyl, hexadecyl, octadecyl, cyclopropyl, cyclobutyl, cyclopentyl or cyclohexyl.

6. Process according to Claims 1 to 5, characterized in that the alkyl carbazate of the formula (II) employed is one in which R² represents methyl or ethyl.

7. Process according to Claims 1 to 6, characterized in that an alkyl carbazate of the formula (II) and an alcohol of the formula (III) are employed in a molar ratio of from 0.5 to 20:1.

8. Process according to Claims 1 to 7, characterized in that the catalysts employed are alkali metal hydroxides, alkaline earth metal hydroxides, alkali metal or alkaline earth metal carbonates or hydrogen carbonates or alkali metal or alkaline earth metal alcoholates, amino compounds, titanium compounds or tin compounds.

9. Process according to Claims 1 to 8, characterized in that crude alkyl carbazates of the formula (II) are employed as are obtainable in the reaction of dialkyl carbonates of the formula (IV)
R²-O-CO-O-R² (IV),
in which R² is as defined in Claim 2 for formula (II), with hydrazine without further purification or intermediate isolation.

10. Process according to Claims 1 to 9, characterized in that the catalyst is employed in amounts of from 0.001 to 10 % by weight, based on the alkyl carbazate of the formula (II), and the reaction is carried out at from 50 to 250°C.

## Revendications

1. Procédé pour la préparation des carbazates de formule (I)
R¹-O-CO-NH-NH₂ (I),
dans laquelle R¹ représente un groupe alkyle à chaîne droite ou ramifiée en C₅-C₁₈, éventuellement substitué par le fluor, le chlore, le groupe X-R³ (avec X = oxygène ou soufre et R³ = alkyle à chaîne droite ou ramifiée en C₁-C₄), un groupe cycloalkyle en C₃-C₆ éventuellement substitué, un groupe aryle en C₆-C₁₂ éventuellement substitué, un groupe alkyle en C₂-C₁₈ substitué par un groupe phényle, un groupe benzyle ou un groupe cyclopropylméthyle,
ce procédé se caractérisant en ce que l'on fait réagir en présence d'un catalyseur un carbazate d'alkyle de formule (II)
R²-O-CO-NH-NH₂ (II),
dans laquelle R² représente un groupe alkyle à chaîne droite ou ramifiée en C₁-C₄, non substitué,
avec un alcool de formule (III)
R¹-OH (III),
dans laquelle R¹ a les significations indiquées en référence à la formule (I).

2. Procédé selon revendication 1, caractérisé en ce que, dans les formules (I) et (III), lorsque R¹ représente un groupe alkyle en C₃-C₆ substitué ou un groupe aryle en C₆-C₁₂ substitué, les substituants sont des halogènes, des groupes hydroxy, le groupe X-R³ (avec X = oxygène ou soufre et R³ = alkyle à chaîne droite ou ramifiée en C₁-C₄), le groupe COOR⁴ (avec R⁴ = alkyle en C₁-C₄), le groupe NR⁵R⁶ (avec R⁵ et R⁶ = indépendamment l'un de l'autre, alkyle en C₁-C₄) et cycloalkyle en C₃-C₆ portant lui-même éventuellement des substituants alkyles à chaîne droite ou ramifiée en C₁-C₄, et lorsqu'il s'agit d'un groupe cycloalkyle en C₃-C₆, les substituants peuvent également consister en groupes aryles en C₆-C₁₄ éventuellement substitués ou aryloxy en C₆-C₁₄ éventuellement substitués, 1-pipéridinyle, 1-morpholinyle et 1-pyrrolidinyle.

3. Procédé selon les revendications 1 et 2, caractérisé en ce que R¹ représente un groupe cycloalkyle en C₃-C₆, éventuellement substitué par le fluor, le chlore, le groupe X-R³ (tel que défini dans la revendication 2) ou le groupe phényle lui-même éventuellement substitué par des groupes alkyles enC₁-C₄, alcoxy en C₁-C₄, le fluor et/ou le chlore.

4. Procédé selon revendication 1, caractérisé en ce que R¹ représente un groupe cycloalkyle en C₃-C₆ éventuellement substitué par un groupe alkyle en C₁-C₂.

5. Procédé selon les revendications 1 à 4, caractérisé en ce que R¹ représente un groupe pentyle, isopentyle, hexyle, dodécyle, hexadécyle, octadécyle, cyclopropyle, cyclobutyle, cyclopentyle, cyclohexyle.

6. Procédé selon les revendications 1 à 5, caractérisé en ce que l'on utilise un carbazate d'alkyle de formule (II), dans laquelle R² représente un groupe méthyle ou éthyle.

7. Procédé selon les revendications 1 à 6, caractérisé en ce que l'on met en oeuvre un carbazate d'alkyle de formule (II) et un alcool de formule (III) à un rapport molaire de 0,5 à 20 : 1.

8. Procédé selon les revendications 1 à 7, caractérisé en ce que l'on utilise en tant que catalyseurs des hydroxydes de métaux alcalins, des hydroxydes de métaux alcalino-terreux, des carbonates ou bicarbonates de métaux alcalins ou alcalino-terreux ou des alcoolates de métaux alcalins ou alcalino-terreux, des dérivés aminés, des dérivés du titane ou des dérivés de l'étain.

9. Procédé selon les revendications 1 à 8, caractérisé en ce que l'on met en oeuvre les carbazates d'alkyle de formule (II) à l'état brut, tels qu'obtenus par réaction de carbonates de dialkyle de formuel (IV)
R²-O-CO-O-R² (IV),
dans laquelle R² a les significations indiquées dans la revendication 2 en référence à la formule (II), avec l'hydrazine, sans purification supplémentaire ni isolement intermédiaire.

10. Procédé selon les revendications 1 à 9, caractérisé en ce que le catalyseur est mis en oeuvre en quantité de 0,001 à 10 % du poids du carbazate d'alkyle de formule (II) et en ce que la réaction est exécutée à des températures de 50 à 250°C.
